Europäisches Patentamt

(19) European Patent Office

Office européen des brevets

(11) EP 0 489 472 B1

## (12) EUROPEAN PATENT SPECIFICATION

(45) Date of publication and mention
of the grant of the patent:
**29.10.1997 Bulletin 1997/44**

(51) Int. Cl.$^6$: **C07C 51/14**

(21) Application number: **91203137.4**

(22) Date of filing: **29.11.1991**

(54) **Carbonylation process and catalyst composition**

Verfahren zur Carbonylierung und Katalysatorzusammensetzung

Procédé de carbonylation et composition catalytique

(84) Designated Contracting States:
**BE DE FR GB IT NL**

(30) Priority: **03.12.1990 GB 9026210**
**03.12.1990 GB 9026211**

(43) Date of publication of application:
**10.06.1992 Bulletin 1992/24**

(73) Proprietor: **SHELL INTERNATIONALE**
**RESEARCH**
**MAATSCHAPPIJ B.V.**
**2596 HR Den Haag (NL)**

(72) Inventors:
• **Drent, Eit**
**NL-1031 CM Amsterdam (NL)**
• **Koenders, Peter**
**NL-1031 CM Amsterdam (NL)**

(56) References cited:
**EP-A- 0 386 834**     **US-A- 3 530 155**
**US-A- 4 414 409**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.14.18/3.4

**Description**

The invention relates to a process for the carbonylation of olefinically or acetylenically unsaturated compounds, and more in particular to such a process conducted in the presence of a nucleophilic compound having one or more removable hydrogen atoms, and a palladium catalyst, and to certain catalyst compositions particularly suited to said process.

Processes for the carbonylation of olefinically unsaturated compounds, hereinafter referred to as olefins, or of acetylenically unsaturated compounds, hereinafter referred to as acetylenes, with carbon monoxide in the presence of a nucleophilic compound containing one or more removable hydrogen atoms, such as for instance an alcohol or a carboxylic acid, and a palladium catalyst are known, and have been described e.g. in EP 0055875, 0106379, 0168876 and 0186228, as are the products which may be prepared by such a process. Generally such processes are conducted by introducing the various reactants into the reactor and allowing the reaction to proceed under the desired reaction conditions. With some of these processes the presence in the reaction medium of a suitable solvent was found to have a beneficial effect, e.g. by promoting the solubility and/or miscibility of the reactants and/or by reducing the viscosity of the reaction medium; the desirability for a solvent to be present as well as the nature of such a solvent being largely determined by the nature of the reactants.

Sometimes the reaction medium of such a process comprises two immiscible liquid phases. This can for instance be the case with the preparation of the full esters of polyhydric alcohols via the carbonylation of olefins in the presence of such a polyhydric alcohol, as the latter are frequently solid compounds having a high melting point and moreover do not dissolve readily or at all in the olefin reactant. Such polyhydric alcohols may then be employed as a solution in a highly polar solvent, which solutions generally result in the formation of a two-phase liquid reaction medium with the olefin reactant. Another example of immiscible reactants, is the use of a very polar liquid reactant, such as a polyethylene glycol or water, which would normally result in a two-phase medium when combined with most of the olefins or acetylenes. On the other hand it is also possible that the two-phase liquid reaction medium has been intentionally created. This would for instance be the case when a liquid compound wherein only one the reactants will dissolve is added to the reaction medium, this non-mutual solvent being present to facilitate the catalyst retrieval at the end of the reaction. The nature of said solvent being governed by the nature of the catalyst system as well as by that of the reaction product.

It has been observed that the presence of a two-phase liquid reaction medium in a process as described hereinbefore, could however result in a reduction of the reaction rate as well as in the degree of conversion, and occasionally in hardly any reaction at all.

In UK Patent Application GB 2 023 589 A a process has been described for the continuous preparation of the full esters of mono- or polypentaerythritol via the carbonylation of olefins having at least 4 carbon atoms, in the presence of such a polyhydric alcohol and a cobalt-based catalyst, and wherein the formation of an inhomogeneous reaction medium can be avoided by a phased addition of the olefin reactant, i.e. initially adding only a part of the olefin reactant to the reactor together with all the polyol and catalyst, and subsequently adding the residual amount of olefin to a middle section of the reactor.

A general disadvantage of said carbonylation process is the use of a cobalt carbonyl catalyst, which catalyst is generally known to have a lower activity and selectivity than a palladium-based catalyst as described e.g. in EP 0106379, and will moreover require more severe reaction conditions, i.e. higher temperatures and/or pressures. A further disadvantage envisaged is, that the relative amounts of olefinic material to be introduced at the beginning and in the middle section of the reactor will vary strongly with the molecular weight of the olefin and the nature of the polyhydric alcohol, and will thus have to be determined separately for each combination of reactants. Finally it does not solve the problem when the two-phase liquid reaction medium has been created intentionally.

US-A-3,530,155 relates to hydrocarboxylation of olefins by contacting the olefin with a catalyst comprising a platinum or palladium complex with an aromatic phosphine, water and carbon monoxide. The phosphines described to be suitable are arylphosphines or mixed alkylarylphosphines, optionally substituted with an alkyl or chloro group. The problems inherent to a multi-phase liquid reaction medium have not been mentioned.

The problem underlying the present invention is developing a process for the carbonylation of olefins or acetylenes in the presence of a nucleophilic compound having one or more removable hydrogen atoms, wherein the combination of said reactants or the presence of one or more selected solvents results in the formation of a reaction medium having > 1 liquid phase, hereinafter referred to as multi-phase reaction medium, and which process does not suffer from one or more of the disadvantages as mentioned hereinbefore.

As a result of extensive research and experimentation a process was developed which enables the carbonylation of olefins or acetylenes with carbon monoxide in the presence of an organic nucleophilic compound having one or more removable hydrogen atoms, in a multi-phase liquid reaction medium, by employing a palladium-based catalyst system which includes selected organic anions.

The invention thus provides a process for the carbonylation of an olefinically or acetylenically unsaturated compound with carbon monoxide in the presence of a nucleophilic compound having a removable hydrogen atom and a catalyst system comprising

a) a source of palladium,

b) a phosphine of general formula $PR^1R^2R^3$, wherein $R^1$, $R^2$ and $R^3$ independently represent an optionally substituted alkyl, cycloalkyl, aryl or N-heterocyclic group, and

c) a source of anions being the conjugated base of an acid having a pKa < 3,

wherein the reaction medium at least initially is a multi-phase liquid reaction medium, and in which the catalyst system comprises a component b) having an amphiphatic structure.

In the context of the present invention the term "the reaction medium is at least initially a multi-phase liquid reaction medium" refers to a reaction medium which is at least a multi-phase liquid reaction medium at the beginning of the reaction and during the early stages thereof. The term "amphiphatic structure" means that such molecules are composed of groups of opposing solubility tendencies, typically an oil-soluble hydrocarbon chain and a water-soluble ionic group.

The source of palladium (catalyst component a) preferably is constituted by cationic compounds such as for example the salts of palladium with, for instance, nitric acid, sulphuric acid or alkanecarboxylic acids having not more than 12 carbon atoms. Moreover, palladium complexes may also be used, for instance palladium acetylacetonate, tetrakis(triphenylphosphine)palladium, bis(tri-o-tolylphosphine)palladium acetate or bis(triphenylphosphine)palladium sulphate. Metallic palladium may be used if the catalyst composition comprises an acid component. Palladium acetate is a preferred palladium compound for the catalyst composition of the present invention to be based upon.

The groups $R^1$, $R^2$ and $R^3$ of the phosphine of general formula $PR^1R^2R^3$ (catalyst component b) each represent an optionally substituted alkyl, cycloalkyl, aryl or N-heterocyclic group. In general such alkyl groups will have up to 20 carbon atoms, any cycloalkyl groups will have from 5-7 carbon atoms in the ring structure and any aryl group will have up to 18 carbon atoms in the ring structure. Conveniently any aryl group may be an anthryl, naphthyl or, which is preferred, a phenyl group. The heterocyclic ring may be a single heterocyclic ring or may be a part of an optionally substituted larger, condensed ring structure as exemplified by pyridyl, pyrazinyl, quinolyl, isoquinolyl, pyrimidyl, pyridazinyl, indolizinyl, cinnolinyl, acridinyl, phenazinyl, phenanthridinyl, phenanthrolinyl, phthalazinyl, naphthyridinyl, quinoxalinyl and quinazolinyl groups.

Preferred substituted phosphines are triphenylphosphines carrying one or more substituted phenyl groups.

The phosphine component b of the catalyst system constitutes the catalyst component having an amphiphatic structure, in which case it has a group $R^1$, $R^2$ or $R^3$ substituted with an ionic group providing water-solubility tendency to the otherwise oil-soluble phosphine ligand. Preferred ionic substituents are sulphonic, phosphonic, phosphinic and carboxylic acid groups and their salts. More preferred substituents are sulphonic acid groups or the alkali metal or ammonium salts thereof, sodium sulphonate type substituents being especially preferred.

Examples of preferred triarylphosphines wherein one or more of groups R carry a specified substituent include sodium 4-(diphenylphosphino)benzenesulphonate, phenyl-bis-(sodium 4-sulphonatophenyl)phosphine. Examples of preferred alkyl(diaryl)phosphines include sodium 2-(diphenylphosphino)ethanesulphonate and sodium 3-(diphenylphosphino)propanesulphonate. These substituted phosphines can be obtained by processes as described in EP-A-280380.

The source of anions (catalyst component c) can be any compound or system which is capable of generating anions of the type as required. Preferably the source of anions is the acid of which the anions are the conjugated base, or a salt of said acid. Preferred salts are alkali or alkaline earth metal salts. Should the anion source be an acid, then said acid can simultaneously act as an acid promoter for the catalyst system.

Acids having a pKa < 3 ((measured in water at 18 °C) and providing anions in the catalyst compositions for use in the process of the invention preferably have a non-coordinating anion, by which is meant that little or no co-valent interaction takes place between the palladium and the anion. Typical examples of such anions are $PF_6$, $SbF_6$, $BF_4$ and $ClO_4$.

Acids preferably used are, for instance, sulphonic acids and those acids that can be formed, possibly in situ, by interaction of a Lewis acid such as, for example, $BF_3$, $AsF_5$, $SbF_5$, $PF_5$, $TaF_5$ or $NbF_5$ with a Brønsted acid such as, for example, a hydrohalogenic acid, in particular HF, phosphoric acid or sulphuric acid. Specific examples of the last-named type of acids are fluorosilicic acid, $HBF_4$, $HPF_6$ and $HSbF_6$. Typical sulphonic acids that can be used are fluorosulphonic acid, chlorosulphonic acid, p-toluenesulphonic acid and trifluoromethanesulphonic acid; the last too acids being preferred.

The anion component c of the catalyst system may have an amphiphatic structure in which case it comprises a hydrophobic moiety providing oil-solubility tendency to the otherwise water-soluble anion. More particularly, the anion may have a hydrocarbyl moiety being free of olefinic and or acetylenic unsaturation and containing at least 10 carbon atoms. Preferred types of acids of which the anions, as described hereinbefore are amphiphatic, are selected from the group of acids consisting of sulphonic acids, phosphonic and phosphinic acids; sulphonic acids being especially preferred.

Very suitable sulphonic acids having at least 10 carbon atoms include alkylsulphonic acids, especially linear alkylsulphonic acids, alkarylsulphonic acids such as alkylbenzenesulphonic acids, alkyltoluenesulphonic acids and alkylxylenesulphonic acids, and hydroxyalkylsulphonic acids e.g. $\alpha$- or $\beta$-hydroxyalkylsulphonic acids.

Sulphonic acids as mentioned hereinbefore are readily available in the form of the corresponding sulphonates, and especially the alkali metal sulphonate, as such compounds are well known for their detergent properties. Frequently such sulphonates occur as a mixture of isomers and/or a mixture of compounds having a slight variation in the number of carbon atoms per molecule. With latter type of sulphonates it is desirable that the average number of carbon atoms per anion is at least 10 and preferably at least 15. In view of its availability as a single compound, sodium 4-octadecyl-p-xylenesulphonate is a preferred sodium sulphonate for use as anion source in the process of the present invention. An example of a suitable mixed sulphonic acid is a 4-($C_{12-15}$ alkyl)-p-xylenesulphonic acid. It was mentioned hereinbefore, when employing a salt as anion source, that the corresponding acid may be obtained therefrom by interaction with the acid promoter, for which it is preferred that the $pK_a$ of the acid promoter $\leq$ the $pK_a$ of the acid on which said salt is based.

In one embodiment of the invention, the catalyst system comprises both an amphiphatic phosphine component b, such as phenyl-bis-(sodium 4-sulphonatophenyl)phosphine and an amphiphatic anion component c, such as sodium 4-octadecyl-p-xylenesulphonate.

The quantity of palladium compound, as used as catalyst component in the process of the invention, is not critical. Preference is given to the use of quantities in the range between $10^{-5}$ and $10^{-1}$ gram atom palladium per mol of olefinically or acetylenically unsaturated compound.

In general the amount of phosphine ligand to be employed in the catalyst composition for use in the process of the present invention is not critical and may vary over wide ranges, which ranges may sometimes also be related to the type of phosphine ligand. For example, diphosphines will generally be employed in a quantity of 0.5-100 mol per gram atom of palladium, and phosphines containing heterocyclic groups as described hereinbefore will generally be employed in a quantity of 2-500 mol per gram atom of palladium, whereas triphenylphosphines are preferably used in a quantity of at least 5 mol per gram atom of palladium. Other monophosphines may conveniently be employed in a quantity in the range of from 0.5-50 mol per gram atom of palladium.

In the process of the present invention it is preferred that the anions, in particular the anions having a hydrocarbyl moiety containing at least 10 carbon atoms, are present in an amount which corresponds with $\geq 2$ eq. of anion per gram atom of palladium.

In the process according to the invention acids may be used as promoters, in particular acids having a non-co-ordinating anion, by which is meant that little or no co-valent interaction takes place between the palladium and the anion. Typical examples of such anions are $PF_6^-$, $SbF_6^-$, $BF_4^-$ and $ClO_4^-$ and the further anions mentioned hereinbefore in connection with the anion component c of the catalyst system.

A special class of acids for use in the process of the present invention are those which can simultaneously act as promoter and as anion source. Hence the anion of such acids may have a hydrocarbyl moiety containing at least 10 carbon atoms and preferably at least 15, and constitute the amphiphatic catalyst component in accordance with the invention. Acids meeting these requirements have been discussed and exemplified hereinbefore. When employing such an acid which can act as promoter and as anion source, the presence of a separate anion source in the present process is not essential. With the process of the present invention it is preferred that the acid promoter is present in an amount which corresponds with $> 2$ equivalents $H^+$ per gram atom of palladium.

The olefinically or acetylenically unsaturated compound may be an unsubstituted or substituted linear, branched or cyclic compound preferably having 2-30, and in particular 2-20, carbon atoms and preferably 1-3 double, respectively triple bonds. The unsaturated compounds may be substituted, for instance, with one or more halogen atoms or cyano, ester, alkoxy, hydroxy, carboxy or aryl groups. If the substituents are not inert under the reaction conditions, the carbonylation reaction may be accompanied with other reactions. Examples of suitable olefinic compounds are ethene, propene, butene-1, butene-2, isobutene, cyclopentenes, the isomeric pentenes, hexenes, octenes and dodecenes, 1,5-cyclooctadiene, cyclododecene, 1,5,9-cyclododecatriene, allyl alcohol, methyl acrylate, ethyl acrylate, methyl methacrylate, acrylonitrile, acrylamide, N,N-dimethyl acrylamide, vinyl chloride, allyl chloride, acrolein, oleic acid, methyl allyl ether and styrene. Examples of suitable acetylenes include propyne, 1-butyne, 2-butyne, 1-pentyne, 1-hexyne, 1-heptyne, 1-octyne, 2-octyne, 4-octyne, 5-methyl-3-heptyne, 4-propyl-2-pentyne, 1-nonyne, phenylethyne and cyclohexylethyne.

The nucleophilic type reactants having one or more removable hydrogen atoms include water, mono- and polyhydric alcohols, such as for example mono- and polypentaerythritol, 1,1,1-trimethylolethane, 1,1,1-trimethylolpropane, hydroquinone, pyrogallol, ethylene glycol and polyethylene glycol, as well as mono- or polycarboxylic acids i.e. aliphatic, cycloaliphatic or aromatic carboxylic acids having e.g. up to 20 carbon atoms and optionally carrying one or more inert substituents such as for example halogen atoms, and cyano, ester, alkoxy and aryl groups. Examples of suitable mono- or polycarboxylic acids include formic acid, acetic acid, propionic acid, n-butyric acid, isobutyric acid, pivalic acid, valeric acid, hexanoic acid, octanoic acid, nonanoic acid, decanoic acid, dodecanoic acid , hexadecanoic acid, octadecanoic acid, benzoic acid, o-,m- and p-phthalic acid and o-,m- and p-toluic acid, adipic acid, glutaric acid and sebacic acid. A further group of potentially suitable nucleophilic compounds of the type as described hereinbefore include compounds such as hydroxysulphonates and primary and secondary amines.

Data regarding the combination of olefins or acetylenes and nucleophilic reactants which will result in a multi-phase liquid reaction medium, under the conditions required for conducting the carbonylation in the process of the present invention, may be obtained from the technical literature, in so far not already known by those skilled in the act. It will be appreciated that not only the nature of the olefin and alcohol reactant per se but also the ratio wherein they are to be employed, may be decisive for the formation of a multi-phase liquid reaction medium.

When employing a nucleophilic reactant, having a relatively high melting point and which does not dissolve in the olefin reactant, such as for example a mono- and polypentaerythritol, 1,1,1-trimethylolethane, hydroquinone and pyrogallol, said compound may be employed as a solution. Suitable solvents which may be employed for the preparation of such solutions include sulfolane, dimethyl sulphoxide and diisopropyl sulphone. It is also possible that the phase containing the olefin or acetylene reactant may contain a solvent or diluent, e.g. to reduce the viscosity thereof, which solvent or diluent will generally be a hydrocarbon type of solvent or diluent.

It was mentioned hereinbefore that the multi-phase liquid reaction medium should at least be present in the early stages of the reaction, as it is conceivable that the multi-phase liquid reaction medium will gradually be converted into a single-phase liquid reaction medium as the reaction progresses and the amount of reaction product increases, thus enabling the reaction to further proceed in a homogeneous liquid reaction medium, and in the presence of the catalyst system as described hereinbefore.

Without wishing to be bound by any theory it is believed that an important feature of the process of the present invention lies in the presence in the catalyst systems of amphiphatic components as specified hereinbefore. It is believed that the observed reduction in reaction rate, when conducting the carbonylation of olefins or acetylenes with carbon monoxide in a multi-phase liquid reaction medium in the presence of nucleophilic reactant and a conventional carbonylation catalyst according to the prior art, is related to a non-uniform catalyst distribution, i.e. the catalyst system being predominantly present in the nucleophilic reactant-containing phase. However, when employing a catalyst system which includes an amphiphatic component, such as an anionically substituted phosphine, the presence of said amphiphatic components will result in the catalyst system being preferentially orientated at the interface of the phases, thereby increasing the reaction rate. Simultaneously the relatively high concentration of catalyst at the interface may also have a beneficial influence on the local miscibility of the phases.

It can furthermore be expected, when the multi-phase liquid reaction medium has been converted to a single-phase or homogeneous liquid reaction medium, that the reaction will proceed as if a conventional catalyst system had been present, i.e. one which does not include a specific phosphine or anion source as described hereinbefore. In fact the catalyst systems as described hereinbefore may conveniently be also employed in a carbonylation process which is conducted in a homogeneous liquid reaction medium.

In the process according to the invention the carbon monoxide may be used pure or diluted with an inert gas, such as nitrogen, noble gases or carbon dioxide. Generally the presence of more than 10 %v of hydrogen is undesirable, since under the reaction conditions it may cause hydrogenation of the olefinic compound. Generally preference is given to use to carbon monoxide or a carbon monoxide-containing gas which contains less that 5 %v of hydrogen.

The carbonylation according to the invention is preferably carried out at a temperature in the range between 50 and 200 °C, in particular between 75 and 150 °C. The overall pressure preferably lies between 1 and 100, in particular 20 and 75 bar gauge.

The process according to the present invention may be carried out batchwise, continuously or semi-continuously.

Catalyst systems comprising a palladium compound, a phosphine of general formula $PR^1R^2R^3$, wherein $R^1$, $R^2$ and $R^3$ independently represent an optionally substituted alkyl, cycloalkyl, aryl or N-heterocyclic group, and a source of anions being the conjugated base of an acid having a pKa < 3, which anions preferably have a hydrocarbyl moiety free of olefinic and/are acetylenic unsaturation and containing at least 10 carbon atoms, in which catalyst system component b) has an amphiphatic structure are novel.

A further novel catalyst system provided by the invention is based on a palladium compound, a phosphine of general formula $PR^1R^2R^3$ , wherein $R^1$, $R^2$ and $R^3$ independently represent an optionally substituted alkyl, cycloalkyl, aryl or N-heterocyclic group, at least one of which carries a substituent selected from the group consisting of sulphonic, phosphonic, phosphinic and carboxylic acid groups, or a salt thereof, and a source of anions having a hydrocarbyl moiety being free of olefinic and or acetylenic unsaturation and containing at least 10 carbon atoms, and being the conjugated base of an acid having a pKa < 3.

The novel catalyst systems may further comprise an acid promoter.

Although a wide range of products may be prepared by the process of the present invention, the nature of said products will be closely related to the nature of the reactants. For example, when employing water, a hydroxy compound, a carboxylic acid or an amine as the nucleophilic reactant, the reaction product will contain respectively an acid, an ester, an anhydride or an amine moiety, which moieties will furthermore be characterized by the presence of an $\alpha$-$\beta$-olefinically unsaturated group when the coreactant is an acetylenically unsaturated compound. This olefinically unsaturated group will be absent when the coreactant is an olefinically unsaturated compound.

The nature of product will not only be governed by the type of reactant but also by the functionality of the reactants and the molar ratio wherein they are employed. The functionality of the nucleophilic reactant is determined by the number of hydrogen atoms which can be removed under the conditions of the present process, whereas the functionality of the olefinically or acetylenically unsaturated compound is determined by the number of such unsaturated groups per molecule.

The compounds prepared according to the process of the present invention may conveniently be isolated from the reaction mixture by known techniques, such extraction or distillation.

A preferred group of compounds to be prepared by the process of the present invention are the full esters of pentaerythritol and especially those esters wherein the hydrocarbylcarbonyl groups contain 3-19 carbon atoms. Such full esters are valuable products for use as lubricants, detergents and plasticizers. Other products which may be prepared by the present process may be used as precursors for the preparation of e.g. fine chemicals.

The invention will be further illustrated with the following examples for which the following information is provided:

Abbreviations: $Pd(OAc)_2$ Palladium acetate PTSA paratoluenesulphonic acid
Phosphine Type D : Phenyl-bis(sodium m-sulphonatophenyl)phosphine
Anion source I : Sodium 4-octadecyl-p-xylenesulphonate

Example I

A solution of 10 mmol of pentaerythritol in 50 ml sulfolane and the indicated amounts of palladium acetate, phosphine, PTSA, anion source and olefin were introduced into a magnetically stirred 250 ml stainless steel (Hastelloy C, trade mark) autoclave. Subsequently the reactor was closed and the air evacuated therefrom, whereupon 60 bar of carbon monoxide was introduced. This was followed by heating the reactor contents to 110 °C. After maintaining the reactor contents at 110 °C for the required period of time, the reactor contents were cooled to 20 °C, and analysed by means of high performance liquid chromatography to establish hydroxyl conversion. In the process, pentaerythritol polyesters with carboxylic acids having one more carbon atom than the precursor olefins are formed; at 100 % hydroxyl conversion the product is constituted by the tetraester, whereas at lower hydroxyl conversion mixtures further containing tri-, di- and monoesters are obtained. The analytical data have been presented in Table 1, which also gives the relevant process details.

From the results presented in Table 1, it can be observed that the presence of the anion source enables a 100 % hydroxyl conversion to be obtained. With the experiments wherein a 100 % conversion was obtained the residence time in the reactor at 110 °C should not be interpreted as being the required reaction time, as it only indicates that the corresponding result was achieved within that time. The actual reaction time could well be considerably shorter.

Table 1

| Example | $Pd(OAc)_2$ mmol | Phosphine | | PTSA mmol | Anion | source | α-olefin | | conv. OH eq. % | Time at 110 °C h |
|---|---|---|---|---|---|---|---|---|---|---|
| | | Type | mmol | | Type | mmol | Type | ml | | |
| I | 0.5 | D | 6 | 4 | I | 4 | $C_8$ | 70 | 100 | 15 |

Example II

Into a 250 ml stainless steel (Hastelloy C) autoclave were introduced 40 ml diethylene glycol dimethyl ether, 10 ml water, 0.1 mmol palladium acetate, 2 mmol paratoluenesulphonic acid, 4 mmol of phenyl-bis-(sodium 4-sulphonatophenyl)phosphine and 20 ml cyclopentene. Subsequently the reactor was closed and the air removed by pressuring/depressuring cycles with carbon monoxide, which was followed by carbon monoxide addition at a pressure of 40 bar and heating to 110 °C. After a reaction time of 5 h the reactor contents were cooled to room temperature (approx. 20 °C) and analyzed via gas liquid chromatography which showed an olefin conversion of 75 % to cyclopentane carboxylic acid with a selectivity of 100 %).

Example III

The procedure of Example II was repeated except that 4 mmol of sodium 4-octadecyl-p-xylenesulphonate was also introduced into the reactor which resulted in an olefin conversion after 5 h reaction of 85 %.

Comparative Experiment

The procedure of Example II was repeated with the exception that the sulphonate-containing phosphine was replaced with 5 mmol of triphenyl-phosphine. After 5 h the olefin conversion was < 5 %.

**Claims**

1. A process for the carbonylation of an olefinically or acetylenically unsaturated compound with carbon monoxide in the presence of a nucleophilic compound having a removable hydrogen atom and a catalyst system comprising

   a) a source of palladium,
   b) a phosphine of general formula $PR^1R^2R^3$, wherein $R^1$, $R^2$ and $R^3$ independently represent an optionally substituted alkyl, cycloalkyl, aryl or N-heterocyclic group, and
   c) a source of anions being the conjugated base of an acid having a pKa < 3,

   in which process the reaction medium is at least initially a multi-phase liquid reaction medium, and in which the catalyst system comprises a component b) having an amphiphatic structure.

2. A process as claimed in claim 1, wherein the catalyst system further comprises a component c) having an amphiphatic structure.

3. A process as claimed in claim 1 or 2, wherein the catalyst component having an amphiphatic structure is an anionic surfactant.

4. A process as claimed in any one or more of claims 1-3, wherein the nucleophilic compound having a removable hydrogen atom is water, an alcohol or a carboxylic acid.

5. A process as claimed in any one or more of claims 1-4, wherein the anions have a hydrocarbyl moiety being free of olefinic and or acetylenic unsaturation and containing at least 10 carbon atoms.

6. A process as claimed in claim 5, wherein the anions are the conjugated bases of acids selected from the group consisting of sulphonic, phosphonic, phosphinic and carboxylic acids having a pKa < 3.

7. A process as claimed in claim 6, wherein the sulphonic acid is an alkylsulphonic acid or an alkarylsulphonic acid.

8. A process as claimed in claim 7, wherein the alkarylsulphonic acid is a $C_{12-20}$ alkyl p-xylenesulphonic acid.

9. A process as claimed in any one or more of claims 1-8, wherein the catalyst system comprises an acid promoter.

10. A process as claimed in any one or more of claims 1-9, wherein the phosphine of general formula $PR^1R^2R^3$ has at least one group $R^1$, $R^2$ or $R^3$ carrying a substituent selected from the group consisting of sulphonic, phosphonic, phosphinic and carboxylic acid groups, or a salt thereof.

11. A process as claimed in claim 10, wherein the phosphine is a triarylphosphine or an alkyl(diaryl)phosphine.

12. A process as claimed in claim 11, wherein the substituents are sulphonic acid groups or the alkali metal or ammonium salt thereof.

13. A process as claimed in claim 12, wherein the substituent is a sodium sulphonate group.

14. A catalyst composition based on

    a) a palladium compound,
    b) a phosphine of general formula $PR^1R^2R^3$, wherein $R^1$, $R^2$ and $R^3$ independently represent an optionally substituted alkyl, cycloalkyl, aryl or N-heterocyclic group, and
    c) a source of anions being the conjugated base of an acid having a pKa < 3,

    in which catalyst system component b) has an amphiphatic structure.

**15.** A catalyst composition according to claim 14, in which the source of anions has a hydrocarbyl moiety being free of olefinic and/or acetylenic unsaturation and containing at least 10 carbon atoms.

**16.** A catalyst composition according to claim 14 or 15, wherein the phosphine of general formula $PR^1R^2R^3$ has at least one group $R^1$, $R^2$ or $R^3$ carrying a substituent selected from the group consisting of sulphonic, phosphonic, phosphinic and carboxylic acid groups, or a salt thereof.

**17.** A catalyst composition as claimed in any one or more of claims 14-16, comprising an acid promoter.

**Patentansprüche**

**1.** Verfahren zur Carbonylierung einer olefinisch oder acetylenisch ungesättigten Verbindung mit Kohlenmonoxid in Anwesenheit einer nukleophilen Verbindung, die ein abtrennbares Wasserstoffatom aufweist, und eines Katalysatorsystems, das

> a) eine Palladiumquelle,
> b) ein Phosphin der allgemeinen Formel $PR^1R^2R^3$, worin $R^1$, $R^2$ und $R^3$ unabhängig voneinander eine gegebenenfalls substituierte Alkyl-, Cycloalkyl-, Aryl- oder N-heterocyclische Gruppe bedeuten, und
> c) eine Anionenquelle, die die konjungierte Base einer Säure mit einem pKa < 3 ist, umfaßt,

worin das Reaktionsmedium wenigstens zu Beginn ein mehrphasiges flüssiges Reaktionsmedium ist und worin das Katalysatorsystem eine Komponente b) umfaßt, die eine amphiphatische Struktur aufweist.

**2.** Verfahren nach Anspruch 1, worin das Katalysatorsystem weiterhin eine Komponente c) mit einer amphiphatischen Struktur umfaßt.

**3.** Verfahren nach Anspruch 1 oder 2, worin die Katalysatorkomponente mit einer amphiphatischen Struktur ein anionisches grenzflächenaktives Mittel ist.

**4.** Verfahren nach einem oder mehreren der Ansprüche 1 bis 3, worin die nukleophile Verbindung mit einem abtrennbaren Wasserstoffatom Wasser, ein Alkohol oder eine Carbonsäure ist.

**5.** Verfahren nach einem oder mehreren der Ansprüche 1 bis 4, worin die Anionen einen von olefinischer und/oder acetylenischer Unsättigung freien Hydrocarbylrest mit einem Gehalt an wenigstens 10 Kohlenstoffatomen umfassen.

**6.** Verfahren nach Anspruch 5, worin die Anionen die konjungierten Basen von Säuren sind, die aus der Gruppe ausgewählt sind, die aus Sulfonsäuren, Phosphonsäuren, Phosphinsäuren und Carbonsäuren mit einem pKa Wert < 3 besteht.

**7.** Verfahren nach Anspruch 6, worin die Sulfonsäure eine Alkylsulfonsäure oder eine Alkarylsulfonsäure ist.

**8.** Verfahren nach Anspruch 7, worin die Alkarylsulfonsäure eine $C_{12-20}$ Alkyl-p-xylolsulfonsäure ist.

**9.** Verfahren nach einem oder mehreren der Ansprüche 1 bis 8, worin das Katalysatorsystem einen Säurepromotor umfaßt.

**10.** Verfahren nach einem oder mehreren der Ansprüche 1 bis 9, worin das Phosphin der allgemeinen Formel $PR^1R^2R^3$ wenigstens eine Gruppe $R^1$, $R^2$ oder $R^3$ aufweist, die einen Substituenten trägt, ausgewählt aus der aus Sulfonsäure-, Phosphonsäure-, Phosphinsäure- und Carbonsäuregruppen bestehenden Gruppe, oder ein Salz hiervon.

**11.** Verfahren nach Anspruch 10, worin das Phosphin ein Triarylphosphin oder ein Alkyl (diaryl) phosphin ist.

**12.** Verfahren nach Anspruch 11, worin die Substituenten Sulfonsäuregruppen oder die Alkalimetall- oder Ammoniumsalze hiervon sind.

**13.** Verfahren nach Anspruch 12, worin der Substituent eine Natriumsulfonatgruppe ist.

8

**14.** Katalysatorzusammensetzung auf der Basis

    a) einer Palladiumquelle,
    b) eines Phosphins der allgemeinen Formel $PR^1R^2R^3$, worin $R^1$, $R^2$ und $R^3$ unabhängig voneinander eine gegebenenfalls substituierte Alkyl-, Cycloalkyl-, Aryl- oder N-heterocyclische Gruppe darstellen, und
    c) einer Anionenquelle, die die konjungierte Base einer Säure mit einem pKa < 3 ist,

in welchem Katalysatorsystem die Komponente b) eine amphiphatische Struktur aufweist.

**15.** Katalysatorzusammensetzung nach Anspruch 14, worin die Anionenquelle einen Hydrocarbylrest enthält, der frei von olefinischer und/oder acetylenischer Unsättigung ist und wenigstens 10 Kohlenstoffatome enthält.

**16.** Katalysatorzusammensetzung nach Anspruch 14 oder 15, worin das Phosphin der allgemeinen Formel $PR^1R^2R^3$ wenigstens eine Gruppe $R^1$, $R^2$ oder $R^3$ umfaßt, die einen Substituenten trägt, der aus der Gruppe ausgewählt ist, die aus Sulfonsäure-, Phosphonsäure-, Phosphinsäure- und Carbonsäuregruppen besteht, oder ein Salz hievon.

**17.** Katalysatorzusammensetzung nach einem oder mehreren der Ansprüche 14 bis 16 mit einem Gehalt an einem Säurepromotor.

## Revendications

**1.** Procédé pour la carbonylation d'un composé oléfiniquement ou acétyléniquement insaturé avec du monoxyde de carbone en présence d'un composé nucléophile comportant un atome d'hydrogène séparable et d'un système catalytique comprenant

    a) une source de palladium,
    b) une phosphine de formule générale $PR^1R^2R^3$, dans laquelle $R^1$, $R^2$ et $R^3$ représentent indépendamment un groupe alkyle, cycloalkyle, aryle ou N-hétérocyclique éventuellement substitué, et
    c) une source d'anions constituée par la base conjuguée d'un acide ayant un pKa < 3,

procédé dans lequel le milieu réactionnel est au moins initialement un milieu réactionnel liquide multiphase, et dans lequel le système catalytique comprend un composant b) ayant une structure amphiphatique.

**2.** Procédé suivant la revendication 1, dans lequel le système catalytique comprend de plus un composant c) ayant une structure amphiphatique.

**3.** Procédé suivant l'une ou l'autre des revendications 1 et 2, dans lequel le composant catalytique ayant une structure amphiphatique est un agent tensioactif anionique.

**4.** Procédé suivant l'une quelconque des revendications 1 à 3, dans lequel le composé nucléophile comportant un atome d'hydrogène séparable est de l'eau, un alcool ou un acide carboxylique.

**5.** Procédé suivant l'une quelconque des revendications 1 à 4, dans lequel les anions comportent un fragment hydrocarbyle exempt d'insaturation oléfinique et/ou acétylénique et contenant au moins 10 atomes de carbone.

**6.** Procédé suivant la revendication 5, dans lequel les anions sont les bases conjuguées d'acides choisis dans le groupe comprenant les acides sulfoniques, phosphoniques, phosphiniques et carboxyliques ayant un pKa < 3.

**7.** Procédé suivant la revendication 6, dans lequel l'acide sulfonique est un acide alkylsulfonique ou un acide alkarylsulfonique.

**8.** Procédé suivant la revendication 7, dans lequel l'acide alkarylsulfonique est un acide alkyl $C_{12\text{-}20}$ p-xylènesulfoni-que.

**9.** Procédé suivant l'une quelconque des revendications 1 à 8, dans lequel le système catalytique comprend un promoteur d'acide.

**10.** Procédé suivant l'une quelconque des revendications 1 à 9, dans lequel la phosphine de formule générale $PR^1R^2R^3$ comporte au moins un groupe $R^1$, $R^2$ ou $R^3$ comportant un substituant choisi dans le groupe comprenant les groupes d'acide sulfonique, phosphonique, phosphinique et carboxylique, ou un sel de ceux-ci.

11. Procédé suivant la revendication 10, dans lequel la phosphine est une triarylphosphine ou une alkyl(diaryl)phosphine.

12. Procédé suivant la revendication 11, dans lequel les substituants sont des groupes d'acide sulfonique ou leurs sels de métal alcalin ou d'ammonium.

13. Procédé suivant la revendication 12, dans lequel le substituant est un groupe sulfonate de sodium.

14. Composition catalytique à base

   a) d'un composé de palladium,
   b) d'une phosphine de formule générale $PR^1R^2R^3$, dans laquelle $R^1$, $R^2$ et $R^3$ représentent indépendamment un groupe alkyle, cycloalkyle, aryle ou N-hétérocyclique éventuellement substitué, et
   c) d'une source d'anions constituée par la base conjuguée d'un acide ayant un pKa < 3,

   système catalytique dans lequel le composant b) a une structure amphiphatique.

15. Composition catalytique suivant la revendication 14, dans laquelle la source d'anions comporte un fragment hydrocarbyle exempt d'insaturation oléfinique et/ou acétylénique et contenant au moins 10 atomes de carbone.

16. Composition catalytique suivant l'une ou l'autre des revendications 14 et 15, dans laquelle la phosphine de formule générale $PR^1R^2R^3$ a au moins un groupe $R^1$, $R^2$ ou $R^3$ comportant un substituant choisi dans le groupe comprenant les groupes d'acide sulfonique, phosphonique, phosphinique et carboxylique, ou un sel de ceux-ci.

17. Composition catalytique suivant l'une quelconque des revendications 14 à 16, comprenant un promoteur d'acide.